# EUROPEAN PATENT APPLICATION

(11) **EP 4 193 916 A1**
(43) Date of publication of application: **14.06.2023**
(21) Application number: 22190467.5
(22) Date of filing: 16.08.2022
(51) Int. Cl.: A61B 5/053, A61B 5/00, A61B 10/00, G16H 50/30, A61B 5/0536

(54) **NONINVASIVE MEDICAL DIAGNOSTICS USING ELECTRICAL IMPEDANCE METRICS AND CLINICAL PREDICTORS**

(30) Priority: 28.10.2021 US 202163273146 P
(71) Applicant: Prolung, Inc. DBA Ioniq Sciences, Salt Lake City, UT 84103 (US)
(72) Inventor: GARFF, Michael A., Salt Lake City (US); ANDREASEN, Natasha, Salt Lake City (US); BRIMHALL, Owen D., South Jordan (US); KELLEY, Cory J., West Point (US)
(74) Representative: Murgitroyd & Company

(57) **Abstract**

Apparatuses, systems, and methods are disclosed for noninvasive medical diagnostics using electrical impedance metrics and clinical predictors. A method includes applying an electrical current to at least one interrogation electrode placed on a surface of a person's body within a Sappey Plexus region of the person's breast. A method includes measuring an electrical impedance of the person's tissue between the at least one interrogation electrode placed within the Sappey Plexus region of the person's breast and a reference electrode. A method includes comparing the measured electrical impedance to previously-captured electrical impedance measurements of corresponding tissue to determine an indication of a presence of a malignant tumor in the person's tissue.

## Description

### FIELD

The subject matter disclosed herein relates to medical diagnosis and more particularly relates to noninvasive medical diagnostics using electrical impedance metrics and clinical predictors.

### BACKGROUND

While cancers are more prevalent for the aged, they affect individuals of all ages. Those lost due to cancer leave not only human trauma, but also social and significant economic costs to families and society at large. Hence, significant research continues to be focused on various sophisticated diagnostic methods and treatment regimens for various cancerous modalities. It is important that diagnosis be made as early as possible for cancer treatments to have a high probability of success, especially in a non-invasive, low-risk manner for patients.

### SUMMARY

Apparatuses, systems, and methods are disclosed for noninvasive medical diagnostics using electrical impedance metrics and clinical predictors. A method, in one embodiment, includes applying an electrical current to at least one interrogation electrode placed on a surface of a person's body within a Sappey Plexus region of the person's breast. A method, in one embodiment, includes measuring an electrical impedance of the person's tissue between the at least one interrogation electrode placed within the Sappey Plexus region of the person's breast and a reference electrode. A method, in one embodiment, includes comparing the measured electrical impedance to previously-captured electrical impedance measurements of corresponding tissue to determine an indication of a presence of a malignant tumor in the person's tissue.

An apparatus for noninvasive medical diagnostics using electrical impedance metrics and clinical predictors, in one embodiment, includes at least one interrogation electrode, a reference electrode, a processor, and a memory that stores code executable by the processor. In one embodiment, the code is executable by the processor to apply an electrical current to the at least one interrogation electrode placed on a surface of a person's body within a Sappey Plexus region of the person's breast. In one embodiment, the code is executable by the processor to measure an electrical impedance of the person's tissue between the at least one interrogation electrode placed within the Sappey Plexus region of the person's breast and the reference electrode. In one embodiment, the code is executable by the processor to compare the measured electrical impedance to previously-captured electrical impedance measurements of corresponding tissue to determine an indication of a presence of a malignant tumor in the person's tissue.

In one embodiment, an apparatus for noninvasive medical diagnostics using electrical impedance metrics and clinical predictors includes means for applying an electrical current to at least one interrogation electrode placed on a surface of a person's body within a Sappey Plexus region of the person's breast. A method, in one embodiment, includes means for measuring an electrical impedance of the person's tissue between the at least one interrogation electrode placed within the Sappey Plexus region of the person's breast and a reference electrode. A method, in one embodiment, includes means for comparing the measured electrical impedance to previously-captured electrical impedance measurements of corresponding tissue to determine an indication of a presence of a malignant tumor in the person's tissue.

### BRIEF DESCRIPTION OF THE DRAWINGS

In order that the advantages of the invention will be readily understood, a more particular description of the invention briefly described above will be rendered by reference to specific embodiments that are illustrated in the appended drawings. Understanding that these drawings depict only typical embodiments of the invention and are not therefore to be considered to be limiting of its scope, the invention will be described and explained with additional specificity and detail through the use of the accompanying drawings, in which:
Figure 1A is a schematic block diagram illustrating one embodiment of a system for noninvasive medical diagnostics using electrical impedance metrics and clinical predictors;
Figure 1B is a schematic block diagram illustrating one embodiment of a system for noninvasive medical diagnostics using electrical impedance metrics and clinical predictors;
Figure 1C is a schematic block diagram illustrating one embodiment of a probe system for noninvasive medical diagnostics using electrical impedance metrics and clinical predictors;
Figure 1D is a schematic block diagram illustrating one embodiment of measurement results for noninvasive medical diagnostics using electrical impedance metrics and clinical predictors.
Figure 2 is a schematic block diagram illustrating one embodiment of an apparatus for noninvasive medical diagnostics using electrical impedance metrics and clinical predictors;
Figure 3 is a schematic block diagram illustrating one embodiment of an electrode garment for noninvasive medical diagnostics using electrical impedance metrics and clinical predictors;
Figure 4 is a schematic flow chart diagram illustrating one embodiment of a method for noninvasive medical diagnostics using electrical impedance metrics and clinical predictors;
Figure 5 is a schematic block diagram illustrating one embodiment of an apparatus for noninvasive medical diagnostics using electrical impedance metrics and clinical predictors;
Figure 6A illustrates one example of visual feedback for locating a probe on a patient's body;
Figure 6B illustrates another example of visual feedback for locating a probe on a patient's body;
Figure 7 is a schematic flow chart diagram illustrating one embodiment of a method for noninvasive medical diagnostics using electrical impedance metrics and clinical predictors;
Figure 8 depicts one embodiment of an impedance measurement device for noninvasive medical diagnostics using electrical impedance metrics and clinical predictors;
Figure 9A is a perspective view of one embodiment of an interrogation electrode tip for noninvasive medical diagnostics using electrical impedance metrics and clinical predictors;
Figure 9B is a perspective bottom view of one embodiment of an interrogation electrode tip for noninvasive medical diagnostics using electrical impedance metrics and clinical predictors;
Figure 9C is a perspective bottom view of one embodiment of an interrogation electrode tip for noninvasive medical diagnostics using electrical impedance metrics and clinical predictors;
Figure 9D is a perspective cut-away view of one embodiment of an interrogation electrode tip for noninvasive medical diagnostics using electrical impedance metrics and clinical predictors; and
Figure 10 is a schematic flow chart diagram illustrating one embodiment of a method for noninvasive medical diagnostics using electrical impedance metrics and clinical predictors.

### DETAILED DESCRIPTION

Reference throughout this specification to "one embodiment," "an embodiment," or similar language means that a particular feature, structure, or characteristic described in connection with the embodiment is included in at least one embodiment. Thus, appearances of the phrases "in one embodiment," "in an embodiment," and similar language throughout this specification may, but do not necessarily, all refer to the same embodiment, but mean "one or more but not all embodiments" unless expressly specified otherwise. The terms "including," "comprising," "having," and variations thereof mean "including but not limited to" unless expressly specified otherwise. An enumerated listing of items does not imply that any or all of the items are mutually exclusive and/or mutually inclusive, unless expressly specified otherwise. The terms "a," "an," and "the" also refer to "one or more" unless expressly specified otherwise.

Furthermore, the described features, advantages, and characteristics of the embodiments may be combined in any suitable manner. One skilled in the relevant art will recognize that the embodiments may be practiced without one or more of the specific features or advantages of a particular embodiment. In other instances, additional features and advantages may be recognized in certain embodiments that may not be present in all embodiments.

These features and advantages of the embodiments will become more fully apparent from the following description and appended claims or may be learned by the practice of embodiments as set forth hereinafter. As will be appreciated by one skilled in the art, aspects of the present invention may be embodied as a system, method, and/or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module," or "system." Furthermore, aspects of the present invention may take the form of a computer program product embodied in one or more computer readable medium(s) having program code embodied thereon.

Many of the functional units described in this specification have been labeled as modules, in order to emphasize their implementation independence more particularly. For example, a module may be implemented as a hardware circuit comprising custom VLSI circuits or gate arrays, off-the-shelf semiconductors such as logic chips, transistors, or other discrete components. A module may also be implemented in programmable hardware devices such as field programmable gate arrays, programmable array logic, programmable logic devices or the like.

Modules may also be implemented in software for execution by various types of processors. An identified module of program code may, for instance, comprise one or more physical or logical blocks of computer instructions which may, for instance, be organized as an object, procedure, or function. Nevertheless, the executables of an identified module need not be physically located together but may comprise disparate instructions stored in different locations which, when joined logically together, comprise the module and achieve the stated purpose for the module.

Indeed, a module of program code may be a single instruction, or many instructions, and may even be distributed over several different code segments, among different programs, and across several memory devices. Similarly, operational data may be identified and illustrated herein within modules and may be embodied in any suitable form and organized within any suitable type of data structure. The operational data may be collected as a single data set or may be distributed over different locations including over different storage devices, and may exist, at least partially, merely as electronic signals on a system or network. Where a module or portions of a module are implemented in software, the program code may be stored and/or propagated on in one or more computer readable medium(s).

The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory ("RAM"), a read-only memory ("ROM"), an erasable programmable read-only memory ("EPROM" or Flash memory), a static random access memory ("SRAM"), a portable compact disc read-only memory ("CD-ROM"), a digital versatile disk ("DVD"), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture ("ISA") instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network ("LAN") or a wide area network ("WAN"), or the connection may be made to an external computer (for example, through the Internet using an Internet Service Provider). In some embodiments, electronic circuitry including, for example, programmable logic circuitry, field-programmable gate arrays ("FPGA"), or programmable logic arrays ("PLA") may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

Many of the functional units described in this specification have been labeled as modules, in order to emphasize their implementation independence more particularly. For example, a module may be implemented as a hardware circuit comprising custom VLSI circuits or gate arrays, off-the-shelf semiconductors such as logic chips, transistors, or other discrete components. A module may also be implemented in programmable hardware devices such as field programmable gate arrays, programmable array logic, programmable logic devices or the like.

Modules may also be implemented in software for execution by various types of processors. An identified module of program instructions may, for instance, comprise one or more physical or logical blocks of computer instructions which may, for instance, be organized as an object, procedure, or function. Nevertheless, the executables of an identified module need not be physically located together but may comprise disparate instructions stored in different locations which, when joined logically together, comprise the module and achieve the stated purpose for the module.

The schematic flowchart diagrams and/or schematic block diagrams in the Figures illustrate the architecture, functionality, and operation of possible implementations of apparatuses, systems, methods, and computer program products according to various embodiments of the present invention. In this regard, each block in the schematic flowchart diagrams and/or schematic block diagrams may represent a module, segment, or portion of code, which comprises one or more executable instructions of the program code for implementing the specified logical function(s).

It should also be noted that, in some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. Other steps and methods may be conceived that are equivalent in function, logic, or effect to one or more blocks, or portions thereof, of the illustrated Figures.

Although various arrow types and line types may be employed in the flowchart and/or block diagrams, they are understood not to limit the scope of the corresponding embodiments. Indeed, some arrows or other connectors may be used to indicate only the logical flow of the depicted embodiment. For instance, an arrow may indicate a waiting or monitoring period of unspecified duration between enumerated steps of the depicted embodiment. It will also be noted that each block of the block diagrams and/or flowchart diagrams, and combinations of blocks in the block diagrams and/or flowchart diagrams, can be implemented by special purpose hardware-based systems that perform the specified functions or acts, or combinations of special purpose hardware and program code.

As used herein, a list with a conjunction of "and/or" includes any single item in the list or a combination of items in the list. For example, a list of A, B and/or C includes only A, only B, only C, a combination of A and B, a combination of B and C, a combination of A and C or a combination of A, B and C. As used herein, a list using the terminology "one or more of' includes any single item in the list or a combination of items in the list. For example, one or more of A, B and C includes only A, only B, only C, a combination of A and B, a combination of B and C, a combination of A and C or a combination of A, B and C. As used herein, a list using the terminology "one of" includes one and only one of any single item in the list. For example, "one of A, B and C" includes only A, only B or only C and excludes combinations of A, B and C. As used herein, "a member selected from the group consisting of A, B, and C," includes one and only one of A, B, or C, and excludes combinations of A, B, and C." As used herein, "a member selected from the group consisting of A, B, and C and combinations thereof' includes only A, only B, only C, a combination of A and B, a combination of B and C, a combination of A and C or a combination of A, B and C.

A method, in one embodiment, includes applying an electrical current to at least one interrogation electrode placed on a surface of a person's body within a Sappey Plexus region of the person's breast. A method, in one embodiment, includes measuring an electrical impedance of the person's tissue between the at least one interrogation electrode placed within the Sappey Plexus region of the person's breast and a reference electrode. A method, in one embodiment, includes comparing the measured electrical impedance to previously-captured electrical impedance measurements of corresponding tissue to determine an indication of a presence of a malignant tumor in the person's tissue.

In one embodiment, the previously-captured electrical impedance measurements of the corresponding tissue comprise electrical impedance measurements of tissue from within the Sappey Plexus region of different people.

In one embodiment, the previously-captured electrical impedance measurements of the corresponding tissue comprise electrical impedance measurements of tissue from within the Sappey Plexus region of the person's other breast.

In one embodiment, the method includes providing the measured electrical impedance to a machine learning model that is trained on previously-measured electrical impedances, risk factors, and patient data for other people who have been diagnosed with benign and malignant tumors to calculate a risk score for the person.

In one embodiment, the method includes receiving mammogram information associated with the person's breast and, in response to determining that the mammogram information indicates a presence of a nodule within the person's breast, inputting the mammogram information into the machine learning to further calculate the risk score for the person based on the measured electrical impedance.

In one embodiment, the method includes periodically updating the person's risk score based on updated electrical impedance measurements and changes in risk factors and patient data to determine an effectiveness of treatment for post treatment monitoring.

In one embodiment, the method includes heating the at least one interrogation electrode to a temperature corresponding to a predefined electrical conductance. In one embodiment, the method includes adjusting a temperature of the at least one electrode according to a controlled heat profile until a stable electrical current is detected between the at least one interrogation electrode and the reference electrode. In one embodiment, the method includes capturing electrical impedance measurements at various temperatures of the controlled heat profile.

An apparatus for noninvasive medical diagnostics using electrical impedance metrics and clinical predictors, in one embodiment, includes at least one interrogation electrode, a reference electrode, a processor, and a memory that stores code executable by the processor. In one embodiment, the code is executable by the processor to apply an electrical current to the at least one interrogation electrode placed on a surface of a person's body within a Sappey Plexus region of the person's breast. In one embodiment, the code is executable by the processor to measure an electrical impedance of the person's tissue between the at least one interrogation electrode placed within the Sappey Plexus region of the person's breast and the reference electrode. In one embodiment, the code is executable by the processor to compare the measured electrical impedance to previously-captured electrical impedance measurements of corresponding tissue to determine an indication of a presence of a malignant tumor in the person's tissue.

In one embodiment, the interrogation electrode is an electrode tip of an electrode probe. In one embodiment, the electrode tip has a disc shape and a substantially smooth surface. In one embodiment, the electrode tip comprises a textured surface, the textured surface of the brass electrode tip comprising a plurality of protrusions, each of the plurality of protrusions having a hexagonal shape.

In one embodiment, the electrode tip made of a material selected from the group comprising brass, silver-silver chloride, gold, and stainless steel. In one embodiment, the code is further executable by the processor to heat the at least one interrogation electrode to a temperature corresponding to a predefined electrical conductance.

In one embodiment, the code is further executable by the processor to adjust a temperature of the at least one electrode according to a controlled heat profile until a stable electrical current is detected between the at least one interrogation electrode and the reference electrode.

In one embodiment, the code is further executable by the processor to capture electrical impedance measurements at various temperatures of the controlled heat profile. In one embodiment, the previously-captured electrical impedance measurements of the corresponding tissue comprise electrical impedance measurements of tissue from within the Sappey Plexus region of at least one of different people and the person's other breast.

In one embodiment, the code is further executable by the processor to provide the measured electrical impedance to a machine learning model that is trained on previously-measured electrical impedances, risk factors, and patient data for other people who have been diagnosed with benign and malignant tumors to calculate a risk score for the person.

In one embodiment, an apparatus for noninvasive medical diagnostics using electrical impedance metrics and clinical predictors includes means for applying an electrical current to at least one interrogation electrode placed on a surface of a person's body within a Sappey Plexus region of the person's breast. A method, in one embodiment, includes means for measuring an electrical impedance of the person's tissue between the at least one interrogation electrode placed within the Sappey Plexus region of the person's breast and a reference electrode. A method, in one embodiment, includes means for comparing the measured electrical impedance to previously-captured electrical impedance measurements of corresponding tissue to determine an indication of a presence of a malignant tumor in the person's tissue.

In general, the subject matter disclosed herein is directed to diagnosing the presence of a malignant or benign tumor in a patient's body, and in particular in the patient's breast, using non-invasive bioimpedance measurements. Measuring electrical properties associated with these physiological changes that occur when cancer is present in the body has the potential to serve as a non-invasive technology that can provide an early predictive diagnosis for patients with breast lesions. One technology that is especially well-suited to detect these changes is electrical bioimpedance (EBI). EBI has shown promise to provide prognostic information for the detection of many cancers including skin, thyroid, liver, cervix, and breast cancers.

Bioimpedance is particularly well suited for the detection of physiological and structural changes in tissue since it is influenced by key parameters such as electrolyte concentration, pH, hydration state, and cell size and number. Hence, bioimpedance can be used to distinguish between different tissue types or to detect pathological changes in tissue. Instrumentation for bioimpedance varies in complexity depending on how subtle the changes one tries to detect. A two-electrode, DC or single frequency system may suffice in cases where the relative changes are sufficiently large. In other cases, one needs to apply more complex electrode systems to focus the measurements on a specific volume inside the body or multifrequency measurements in a particular frequency range to monitor certain dispersion mechanisms.

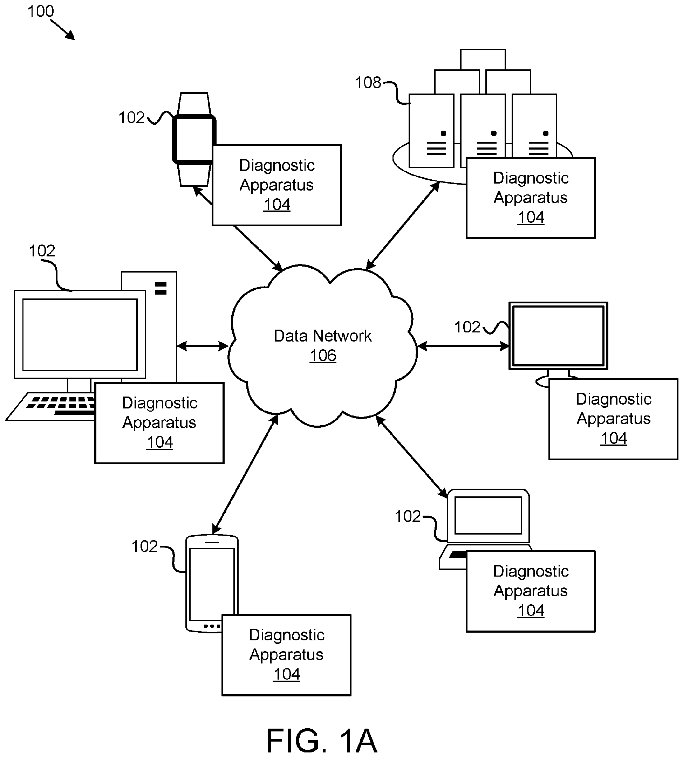

Figure 1A is a schematic block diagram illustrating one embodiment of a system 100 for noninvasive medical diagnostics using electrical impedance metrics and clinical predictors. In one embodiment, the system 100 includes one or more information handling devices 102, one or more diagnostic apparatuses 104, one or more data networks 106, and one or more servers 108. In certain embodiments, even though a specific number of information handling devices 102, diagnostic apparatuses 104, data networks 106, and servers 108 are depicted in Figure 1A, one of skill in the art will recognize, in light of this disclosure, that any number of information handling devices 102, diagnostic apparatuses 104, data networks 106, and servers 108 may be included in the system 100.

In one embodiment, the system 100 includes one or more information handling devices 102. The information handling devices 102 may be embodied as one or more of a desktop computer, a laptop computer, a tablet computer, a smart phone, a smart speaker (e.g., Amazon Echo^{®}, Google Home^{®}, Apple HomePod^{®}), an Internet of Things device, a security system, a set-top box, a gaming console, a smart TV, a smart watch, a fitness band or other wearable activity tracking device, an optical head-mounted display (e.g., a virtual reality headset, smart glasses, head phones, or the like), a High-Definition Multimedia Interface ("HDMI") or other electronic display dongle, a personal digital assistant, a digital camera, a video camera, or another computing device comprising a processor (e.g., a central processing unit ("CPU"), a processor core, a field programmable gate array ("FPGA") or other programmable logic, an application specific integrated circuit ("ASIC"), a controller, a microcontroller, and/or another semiconductor integrated circuit device), a volatile memory, and/or a non-volatile storage medium, a display, a connection to a display, and/or the like.

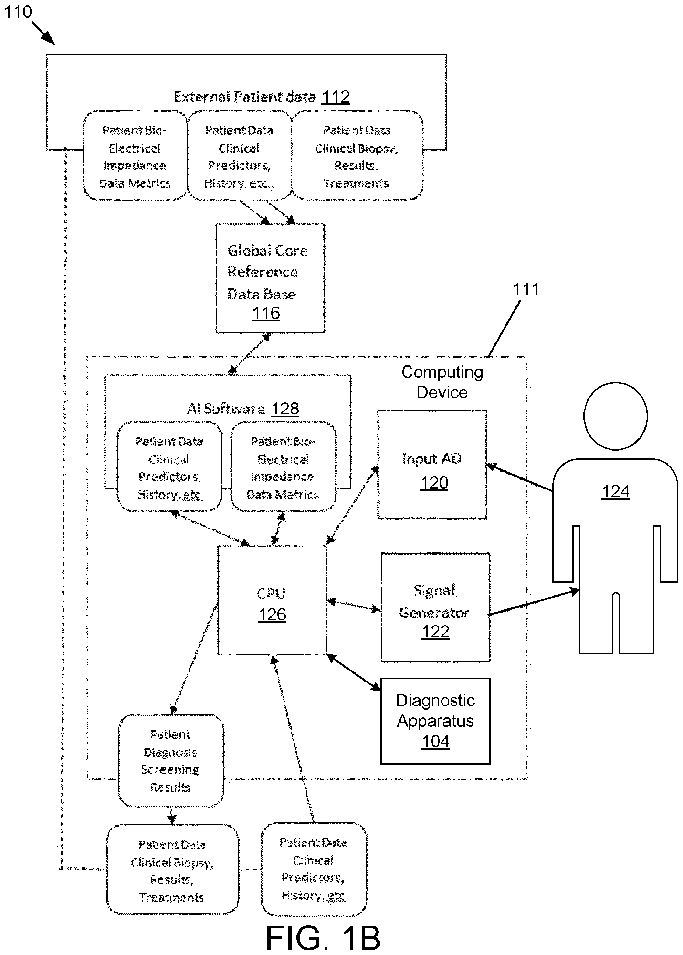

In general, in one embodiment, the diagnostic apparatus 104 is configured to apply, noninvasively, an electrical current to a tissue of a patient's body using an interrogation electrode of a probe. The probe, as shown in Figure 1B, is configured to measure electrical impedance of the tissue between the interrogation electrode and a reference electrode. Furthermore, the diagnostic apparatus 104, in one embodiment, is configured to measure electrical impedance of the tissue of the patient's body between the interrogation electrode of the probe and the reference electrode and detect a presence of a malignant tumor in the tissue of the patient's body by inputting the measured electrical impedance of the tissue into machine learning, which may be trained on patient data associated with a type of disease that is being diagnosed.

In this manner, the diagnostic apparatus 104 detects malignant tumors, e.g., associated with breast or lung cancer, in a non-invasive and non-radiating manner, and in the earliest stages of the tumors, using machine learning to detect, predict, forecast, or the like the presence of a malignant tumor in the patient's body. The diagnostic apparatus 104 uses a combination of bioimpedance measurements, biomarkers, symptoms, risks, and/or the like, together with artificial intelligence, to provide early detection of malignant tumors in the patient, which can increase the survivability of a disease associated with the malignant tumors.

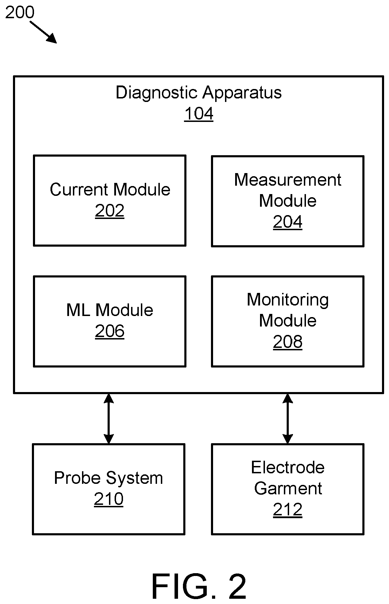

In one embodiment, at least a portion of the diagnostic apparatus 104 is located on an information handling device 102, a probe system, a server 108, an electrode garment (described below), and/or the like. The diagnostic apparatus 104, including its various sub-modules, may be located on one or more information handling devices 102 in the system 100, one or more servers 108, one or more network devices, and/or the like. The diagnostic apparatus 104 is described in more detail below with reference to Figure 2.

In certain embodiments, the diagnostic apparatus 104 may include a hardware device such as a secure hardware dongle or other hardware appliance device (e.g., a set-top box, a network appliance, or the like) that attaches to a device such as a head mounted display, a laptop computer, a server 108, a tablet computer, a smart phone, a security system, a network router or switch, or the like, either by a wired connection (e.g., a universal serial bus ("USB") connection) or a wireless connection (e.g., Bluetooth^{®}, Wi-Fi, near-field communication ("NFC"), or the like); that attaches to an electronic display device (e.g., a television or monitor using an HDMI port, a DisplayPort port, a Mini DisplayPort port, VGA port, DVI port, or the like); and/or the like. A hardware appliance of the diagnostic apparatus 104 may include a power interface, a wired and/or wireless network interface, a graphical interface that attaches to a display, and/or a semiconductor integrated circuit device as described below, configured to perform the functions described herein with regard to the diagnostic apparatus 104.

The diagnostic apparatus 104, in such an embodiment, may include a semiconductor integrated circuit device (e.g., one or more chips, die, or other discrete logic hardware), or the like, such as a field-programmable gate array ("FPGA") or other programmable logic, firmware for an FPGA or other programmable logic, microcode for execution on a microcontroller, an application-specific integrated circuit ("ASIC"), a processor, a processor core, or the like. In one embodiment, the diagnostic apparatus 104 may be mounted on a printed circuit board with one or more electrical lines or connections (e.g., to volatile memory, a non-volatile storage medium, a network interface, a peripheral device, a graphical/display interface, or the like). The hardware appliance may include one or more pins, pads, or other electrical connections configured to send and receive data (e.g., in communication with one or more electrical lines of a printed circuit board or the like), and one or more hardware circuits and/or other electrical circuits configured to perform various functions of the diagnostic apparatus 104.

The semiconductor integrated circuit device or other hardware appliance of the diagnostic apparatus 104, in certain embodiments, includes and/or is communicatively coupled to one or more volatile memory media, which may include but is not limited to random access memory ("RAM"), dynamic RAM ("DRAM"), cache, or the like. In one embodiment, the semiconductor integrated circuit device or other hardware appliance of the diagnostic apparatus 104 includes and/or is communicatively coupled to one or more non-volatile memory media, which may include but is not limited to: NAND flash memory, NOR flash memory, nano random access memory (nano RAM or "NRAM"), nanocrystal wire-based memory, silicon-oxide based sub-10 nanometer process memory, graphene memory, Silicon-Oxide-Nitride-Oxide-Silicon ("SONOS"), resistive RAM ("RRAM"), programmable metallization cell ("PMC"), conductive-bridging RAM ("CBRAM"), magneto-resistive RAM ("MRAM"), dynamic RAM ("DRAM"), phase change RAM ("PRAM" or "PCM"), magnetic storage media (e.g., hard disk, tape), optical storage media, or the like.

The data network 106, in one embodiment, includes a digital communication network that transmits digital communications. The data network 106 may include a wireless network, such as a wireless cellular network, a local wireless network, such as a Wi-Fi network, a Bluetooth^{®} network, a near-field communication ("NFC") network, an ad hoc network, and/or the like. The data network 106 may include a wide area network ("WAN"), a storage area network ("SAN"), a local area network ("LAN") (e.g., a home network), an optical fiber network, the internet, or other digital communication network. The data network 106 may include two or more networks. The data network 106 may include one or more servers, routers, switches, and/or other networking equipment. The data network 106 may also include one or more computer readable storage media, such as a hard disk drive, an optical drive, non-volatile memory, RAM, or the like.

The wireless connection may be a mobile telephone network. The wireless connection may also employ a Wi-Fi network based on any one of the Institute of Electrical and Electronics Engineers ("IEEE") 802.11 standards. Alternatively, the wireless connection may be a Bluetooth^{®} connection. In addition, the wireless connection may employ a Radio Frequency Identification ("RFID") communication including RFID standards established by the International Organization for Standardization ("ISO"), the International Electrotechnical Commission ("IEC"), the American Society for Testing and Materials^{®} (ASTM^{®}), the DASH7^{™} Alliance, and EPCGlobal^{™}.

Alternatively, the wireless connection may employ a ZigBee^{®} connection based on the IEEE 802 standard. In one embodiment, the wireless connection employs a Z-Wave^{®} connection as designed by Sigma Designs^{®}. Alternatively, the wireless connection may employ an ANT^{®} and/or ANT+^{®} connection as defined by Dynastream^{®} Innovations Inc. of Cochrane, Canada.

The wireless connection may be an infrared connection including connections conforming at least to the Infrared Physical Layer Specification ("IrPHY") as defined by the Infrared Data Association^{®} ("IrDA"^{®}). Alternatively, the wireless connection may be a cellular telephone network communication. All standards and/or connection types include the latest version and revision of the standard and/or connection type as of the filing date of this application.

The one or more servers 108, in one embodiment, may be embodied as blade servers, mainframe servers, tower servers, rack servers, and/or the like. The one or more servers 108 may be configured as mail servers, web servers, application servers, FTP servers, media servers, data servers, web servers, file servers, virtual servers, and/or the like. The one or more servers 108 may be communicatively coupled (e.g., networked) over a data network 106 to one or more information handling devices 102, e.g., as part of a healthcare information and patient data system.

Figure 1B depicts a system 110 for noninvasive medical diagnostics using electrical impedance metrics and clinical predictors. As described above, the subject matter herein describes using electrical impedance characterization of biological regions based on normal vs. abnormal levels, combined with clinical predictors and using artificial intelligence/machine learning algorithms to provide predictive screening and detection of malignant tumors, e.g., for various types of cancers or other diseases.

In general, when a malignancy presents itself in the body, the body reacts, e.g., cancers change the composition of the components found in the extracellular matrix. The diagnostic apparatus 104 uses, interfaces with, is in communication with, directs, commands, signals, and/or the like, a probe system to introduce electrical current to predetermined cancer specific points on the body, measures the ion current through the body, and provides the measurements to an artificial intelligence/machine learning engine, which, together with other biomarkers, biometrics, biohistory, biopsy data, lifestyle data, symptoms, health risks, and other biomics associated with the patient, analyzes the measurements to detect the presence of a malignant tumor in the patient, e.g., in a lung or a breast.

In one embodiment, the diagnostic apparatus 104 measures the conductive or resistive response of the ionic current flowing primarily through the interstitial fluid within the extracellular matrix. In certain embodiments, the diagnostic apparatus 104 not only measures tissue at specific locations, but also measures the bulk resistive changes to the interstitial fluids within the extracellular matrix and lymph systems in key targeted locations on the body. Biological changes within the interstitial and extracellular matrix and lymph systems have been shown to be significant and measurable due to the presence of cancer in the body by various analytical methods.

Generally, electrical current can flow in different ways. Electrical current occurs when charged particles move. Electrons are charged particles. Most people are familiar with electrical current flowing in a conductor such as copper. Common examples are electrical wiring in a home, or a computer. Electrical current can also flow in a vacuum or air as in a vacuum tube, or as an electrical spark or lightning. It can also flow in a plasma, or it can flow as electromagnetic wave such as a radio wave, or as a flow of ions. Most importantly in biology and medicine we are concerned with the electric currents due to the flow of ions. Ions are charged atoms, molecules, or molecular structures such as DNA, or even larger proteins, etc., that are dissolved or suspended in water. The charge on these ions is due to an excess or shortage of electrons. Electrical current can flow through a liquid (such as in a human body). This current is made up of the movement of ions. Ions can be negatively charged or positively charged and will flow in opposite directions.

The electrical current flowing through the body is due to a process of ionic flow. This is true for direct current ("DC") or for alternating currents ("AC"). When a voltage is applied to a body or tissue, an electrical current will flow. The electricity only flows as charged ions move toward the oppositely charged electrodes. This is often referred to as bioconductance. The movement of ions is resisted by a number of factors in the body. The resistance to electrical current flow can be referred as an electrical impedance. The electrical impedance can further be characterized as a function of electrical resistance and electrical capacitance. The electrical impedance can be described by a set of complex variables depending on the type of voltage signal applied. The impedance in the body is a function of the frequency of the voltage applied, and the makeup of the biological structures through which it flows.

The diagnostic apparatus 104 improves upon conventional screening systems to increase the accuracy of cancer screening devices that use electrical impedance measurements to diagnose or screen for cancer or other disease states. In certain embodiments, the diagnostic apparatus 104 is further configured to measure the electrical impedances of tissues of interest, anatomical features, interstitial fluids, the lymphatic systems, and/or the like, within the body and provide comparisons with data from known healthy subjects in order to expedient diagnosis and/or screen for detection of disease states including screening for cancers. This includes the electro-impedance characterization of the interstitial fluids in the extracellular spaces, lymph capillaries, lymph channels, lymph nodes, and anatomical tissues of interest. The electrical currents induced and measured in biological systems are based on ion transport. These ionic currents are complex in nature and are dependent on many variables including the ion types, ion concentrations, the matrix through which the ions are moving, and/or the like.

The lymphatic system is considered the immune system of the body. Along with its function as the circulatory system for interstitial fluids it also has functions that modulate of mast cells, T-cells, miRNA, electrolytes, and biochemically significant fragments, etc., that are correlated with cancers, disease states, and various systemic inflammations. Major lymph channels flow along known anatomical areas, corresponding often to intra-fascial plains. The location of lymph nodes is well known including the cervical lymph nodes, axillary lymph nodes, etc. Interstitial fluids in the extracellular spaces have been shown to have substantially higher concentrations of electrolytes, proteins, miRNA, chemokines, and cytokines, related to immune responses, than normal blood.

Measuring electrical impedances of body tissues non-invasively may be a quick way to characterize normal healthy tissue verses unhealthy or abnormal tissues. Clinical studies have shown the efficacy of measuring bioimpedances and their relationship to disease states including various cancers. By increasing the accuracy of the screening devices, one will increase detection capabilities including getting higher sensitivity, and improving specificity. Thus, helping get treatment earlier for some and reducing unneeded treatment for others.

Electrical impedance measurements of the body and tissues provide significant information that has been used to characterize the tissues and fluids of interest. The electrical impedance measurements are complex and provide a nonlinear functions that are voltage, frequency, path, electrode, and tissue dependent. A first order use of the data has led to useful diagnostic information. Adding in the use of artificial intelligence/machine learning, such as deep learning, for signal analysis and comparison to sets of known disease states combined with clinical predictors, such as biomarkers such as, age, sex, weight, height, ethnicity, genomic attributes, blood panel work, medications, location, career, dietary habits, alcohol consumption, family history, income, biopsy results, etc. Combined large sets of data comparing those with known health or disease state and the electrical impedance metrics provide additional predictive power to the noninvasive electrical impedance screening devices. Accurate predictions could be used to reduce the use of unnecessary or risky procedures or expedite there use when appropriate leading to earlier interventions.

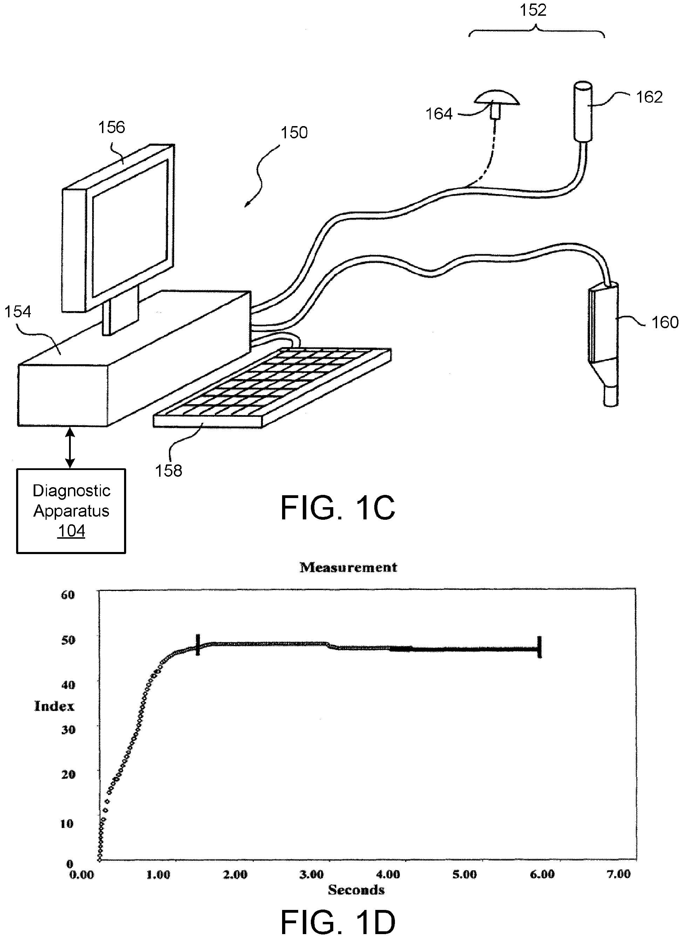

As shown in Figure 1B, a system 110 includes a device 111 to measure electrical impedances through specific areas of the patient's body 124. The device 111 may be a computing device such as a desktop computer, a laptop computer, a mobile device, or any specially-programmed or -configured hardware and software device that includes a processor 116, memory, storage, network capabilities, a display, and/or the like. The device 111 may be a probe system (see Figure 1C), may be communicatively coupled to a probe system, and/or the like.

For instance, the device 111 may include or be communicatively coupled to an input analog-to-digital converter 120 to process signals that are received from electrodes placed on the patient 124 in response to an electrical signal being applied to the patient's body via a probe. The device 111 may further include a signal generator 122 to generate or trigger signals for electrodes that are placed on the patient 124, at various dynamically-determined or predefined voltages, the effect of which is then received by the input AD 120.

Electrodes may be placed on areas of the body that have a correlation with the disease states of interest. These diseases can include various cancers including lung, skin, breast, thyroid, prostate, etc. The system 110 may include means of applying electrical signals as inputs, using the signal generator 122, e.g., using a probe, and the ability to accurately measure and record signals to reference electrodes, including complex impedances, using the input AD 120.

In addition, the system 110 may include a diagnostic apparatus 104 that utilizes artificial intelligence/machine learning algorithms 128 to process and compare the measured signals from specific locations on the patient 124 against a database 116 of signals measured at the same body locations on various subjects with known health states. The database 116 may include external patient data 112 including patient bio-electrical impedance metrics, clinical data such as a predictors and medical history, clinical biopsy results, treatment data, medical informatics information such as age, sex, ethnicity, weight, height, health conditions, medications, smoking history, eating habits, alcohol consumption, income, geographic location, biopsy results, etc. The database 116 may have the ability to grow as more data is included, as more patients are diagnosed, treated, and/or the like.

The database 116 may comprise any type of data store, e.g., a relational database, and may be stored locally or remotely such as in the cloud. The external patient data 112 may be accessed from publicly available patient data (which may have personal identifiable information striped or redacted from the data), data that is provided by hospitals, doctors, clinics, patients, and/or the like. The database 116 may have means of controlling or protecting the core diagnostic data set for diagnostic purposes, e.g., using security measures such as encryption, requiring credentials (username/password, biometric information, etc.), and/or the like.

The diagnostic apparatus 104, in one embodiment, uses the artificial intelligence/machine learning algorithms 118 to do pattern recognition on the data sets - the instant patient data and the external patient data 112 - combining the electrical impedance measurements to provide higher confidence diagnostic scoring. As used herein, artificial intelligence may refer to the ability of a machine/computer to learn over time and simulate intelligent behavior. Furthermore, machine learning, as used herein, may refer to an application of artificial intelligence (AI) that provides systems the ability to automatically learn and improve from experience without being explicitly programmed. Machine learning focuses on the development of computer programs that can access data and use it learn for themselves.

The artificial intelligence/machine learning algorithms 118 may comprise various types of machine learning algorithms such as supervised machine learning algorithms (e.g., nearest neighbor, naive bayes, decision trees, linear regression, support vector machines, neural networks, etc.), unsupervised machine learning algorithms (e.g., k-means clustering, association rules, etc.), semi-supervised machine learning algorithms, and/or reinforcement machine learning algorithms (e.g., Q-learning, temporal difference, deep adversarial networks, etc.).

The diagnostic apparatus 104 may train the artificial intelligence/machine learning 118 on the external patent data in the reference database 116. The diagnostic apparatus 104 may continuously train and/or retrain the artificial intelligence/machine learning as the size the of training data set grows, which provides statistically improved diagnostics scores due to the increased accuracy of the artificial intelligence/machine learning 118. In some embodiments, the results, predictions, estimations, forecasts, diagnostics, and/or the like from the artificial intelligence/machine learning 118 are provided, input, or stored in the database 116 for future reference and training. In certain embodiments, periodic clinical and regulatory review, e.g., by third parties, of the data stored in the database 116, including proposed additions to or improvements to the database 116, may be performed to authorize upgrades to the core diagnostic data set in the database 116.

In one example embodiment, the system 110 includes a device 111 to measure electrical impedances through specific areas of the body located along lymphatic channels and lymph nodes. Test metrics may be correlated with the disease states of interest compared to large statistically significant metrics from known healthy subjects. These states can include various cancer types that can be identified by monitoring the lymphatic system including lung, skin, breast, thyroid, etc. The system 110 includes means of applying controlled electrical signals as inputs at designated locations along the lymphatic system and the ability to accurately measure and record precise signals to reference electrodes, including complex impedances. The electrodes can include single point as well as arrays of specifically spaced electrodes to scan a zone or zones, e.g., a lymphatic node or vessel/channel.

Figure 1C depicts one embodiment of a device for generating electrical current and measuring impedance in a patient's body. In one embodiment, the device includes a computer assembly, generally indicated at 150, and a probe system, generally indicated at 152. The computer assembly 150 typically includes a housing 154 to contain a processor and memory in communication with a display device, such as monitor 156, One or more input device, such as illustrated keyboard 158, a mouse, or the like, may also be included in operable association with the computer assembly. Similarly, an output device, such as a printer, USB port, network connector, media writer, and the like, may be disposed in operable relation with a computer system 150.

The probe system 152 typically includes an interrogation electrode 160. One example of an interrogation electrode that may be used is disclosed in United States Patent Application Publication No. 2005/0015017, published Jan. 20, 2005, the entire contents of which are hereby incorporated by this reference. Desirably, an interrogation electrode 160 will be structured to permit computer controlled application of electrode contact pressure force onto a subject's skin during a measurement sequence. Such computer control may include a feedback loop encompassing real-time conductivity data as measured by the probe itself.

Probe system 152 also includes a reference electrode, such as cylinder 162 that may be hand-held by a subject, or optional spot-probe 164 that may be applied by the clinician. Desirably, the reference electrodes are structured to contact a relatively larger area of a measured subject's skin and isolate the operator from the formed electrical circuit. An operable electrode 162 includes a cylindrical mass of conductive material, such as metal, sized about one inch in diameter, and about three inches in length. Brass is an operable metal from which to form a reference electrode, although other metals and conductive materials are also operable. An operable spot-probe 164 may be formed as a blunt, generally mushroom-shaped, mass of conductive material, such as metal, including brass. The electrodes are placed into electrical communication with conductivity measuring equipment that may conveniently be contained in housing 154 for communication of electrical conductivity data to the computer system 150.

Water is typically sprayed on a subject's hand, and the cylindrical reference electrode 162 is held in the moistened palm of a clenched fist. Sometimes, a strap may be applied to ensure the hand does not inadvertently open or lose contact with the electrode 162. The round reference electrode 164 is placed in specific locations on the back of the subject by the operator using moisture. Other electrodes may be placed on the patient's body (e.g., adhered to the patient's skin or placed against the body as part of a garment, described below). The operator handles the electrode with gloves to maintain electrical isolation and applies uniform pressure during the measurement.

Data acquisition includes measuring conductivity as a function of time, and over a period of time, between a reference electrode disposed at one or more reference point, and an interrogation electrode disposed, typically, at each of a plurality of interrogation points. Certain interrogation point locations that may be operable for use in detecting cancers such as lung or breast cancer are located on the arms, upper arms, shoulder, chest, and back. In some embodiments, the reference electrode 162 will be held in the subject's hand on an opposite side of the body midline from the interrogation point during data acquisition for detection of lung cancer.

In one embodiment, software running on the computer system 150 and/or communicatively coupled to the computer system 150, e.g., a diagnostic apparatus 104 located on the computer system 150 and/or connected to the computer system 150 over a data network 106, is programmed to assist an operator during data acquisition using the probe system 152. For example, the display 156 may present a visual anatomical schematic having a highlighted interrogation point overlay that helps the device operator identify and place the interrogation probe 160.

The screen image may update or change to inform the operator of the desired interrogation point for each point of interest during a data acquisition series. A user-perceptible output, such as a low level modulated tone, may be produced to provide real-time feedback to the device operator to verify completion of an acceptable measurement. The conductance measurement profile for each conductance measurement may be displayed visually on the monitor 156, e.g., the conductance value may be sampled 25 times per second during each conductivity measurement.

Further, the diagnostic apparatus 104 may control probe pressure to insure accurate and consistent measurements. Thus, the pressure applied to the skin surface during operation of the probe is reproducible and independent of operator force. The diagnostic apparatus 104 implements threshold curves during interrogation electrode tip contact that adjusts probe pressure in real-time to assure accurate readings and to prevent erroneous readings. After the measurement session is completed, the diagnostic apparatus 104 may store the data for post processing.

A representative plot of a data-set obtained during time-based measurement of conductivity at an interrogation point is presented in Figure 1D. In Figure 1D, the x-axis represents time, and the y-axis represents measured conductivity index. As used herein, conductivity index is defined as measured conductance equivalent to resistance from 1K ohms to 999K ohms at a nominal 1.2 or 2.4 volts. Firmware in the device 150, e.g., the diagnostic apparatus 104, holds current steady, for example, at 10 microamps, measures the voltage, and then calculates the conductance. The software/firmware of computer system 150, e.g., the diagnostic apparatus 104, employs an algorithm that increases probe pressure until the conductivity index shows a zero slope.

The algorithm then commands constant probe pressure for a period of time, such as for five seconds. In one example use of the electrical interrogation probe, the drop pulse width modulation ("PWM") rate variable of the computer algorithm is set to zero, which keeps the nominal pressure at the electrode tip constant after zero slope is reached. Electrical conductivity is measured between the interrogation probe and reference probe periodically during a time interval and stored as a data-set, and this information is transmitted to the computer system 150. The measured conductance is plotted as the conductivity index normalized on a scale of 0 to 100.

Eight attributes that may be parsed from a data-set, such as that illustrated in Figure 1D, and which describe certain portions of such plot are defined as follows: Base Max is the maximum conductivity index value after zero slope is attained; Base Min is the minimum conductivity index value after zero slope is attained; Rise is the angle between the starting conductivity index and the conductivity index at zero slope; Fall is the angle between the conductivity index at the zero slope point and the conductivity index at the end of measurement; Drop is the difference between the Base Max and the Base Min; Area under the curve to zero slope is the percentage of the area under the curve from start to zero slope as compared to the total possible area from start to zero slope; Area under the curve from zero slope is the percentage of the area under the curve from zero slope to end of measurement as compared to the total possible area from zero slope to end of measurement; and Area under the curve total is the percentage of the area under the curve from start of measurement to end of measurement as compared to the total possible area from start of measurement to end of measurement.

Acceptability of measurements may be determined by the diagnostic apparatus 104 of the system 150, and the clinician may receive perceptible feedback from the computer system 150 to confirm satisfactory completion of a data collection operation. Factors that may be evaluated to determine if data is collected successfully include: 1) Rise in conductivity to a zero slope, computer control. 2) Continued signal measurement thru the sustain timeout value without unexpected fluctuations, computer control and operator control. 3) If an indication of zero slope does not appear within the first two seconds, the measurement should be repeated, operator control. 4) Excessive drop values repeated to confirm, operator control.

Failed measurements may include: 1) Premature zero slope-machine control. 2) Excessive rise or drop after zero slope-machine control. 3) Low conductivity measurement as first measure especially if no other low conductivity measurements operator control. 4) No probe reset at first contact-operator control.

Figure 2 depicts one embodiment of an apparatus 200 for noninvasive medical diagnostics using electrical impedance metrics and clinical predictors. In one embodiment, the apparatus 200 includes an embodiment of a diagnostic apparatus 104. In one embodiment, the diagnostic apparatus 104 includes one or more of a current module 202, a measurement module 204, an ML module 206, and a monitoring module 208. In some embodiments, a probe system 210 and an electrode garment 212 are communicatively coupled to the diagnostic apparatus 104.

In one embodiment, the current module 202 is configured to apply, noninvasively, an electrical current to a tissue of the patient's body using an interrogation electrode of a probe of a probe system 210, as described above. In one embodiment, the probe system 210 may be substantially similar to the probe system 152 described with reference to Figure 1C. The current module 202 may generate a voltage to apply an amount of electrical current in response to an operator's instructions, based on predefined settings related to the type of disease that is being diagnosed, and/or the like.

In one embodiment, the measurement module 204 is configured to measure the electrical impedance of the tissue of the patient's body between the interrogation electrode of the probe and the reference electrode. In certain embodiments, the probe of the probe system 210 may be configured to detect and measure electrical impedance between the interrogation and the reference electrode, or multiple reference electrodes, and store the measurements for use in later analysis and processing.

In one embodiment, the ML module 206 is configured to detect a presence of a malignant tumor in the tissue of the patient's body by inputting the measured electrical impedance of the tissue into machine learning. As described above, the ML module 206 may access, reference, lookup, retrieve, and/or the like measured electrical impedances for the patient and the area of the patient's body that is being examined, and provide the measured electrical impedances to an artificial intelligence/machine learning engine for determining or detecting the presence of a malignant tumor in the tissue where the electrical current is applied.

In such an embodiment, the machine learning is trained on patient data associated with a type of disease that is being diagnosed, e.g., lung cancer, breast cancer, prostate cancer, and/or the like. For example, a machine learning model may be trained to detect lung cancer generally and/or at a particular location of the lung using historical training data that includes electrical impedances that were measured from other patients who have been diagnosed with lung cancer.

In one embodiment, the machine learning model may be trained on other external data such as biomarker data. As used herein, biomarker may refer to a measurable indicator of some biological state or condition, e.g., age, sex, weight, height, ethnicity, genomic attributes, blood panel work, medications, location, career, dietary habits, alcohol consumption, family history, income, previous biopsy results, and/or the like. Digital biomarkers, e.g., biomarkers that are focused on vital parameters such as accelerometer data, heartrate, blood pressure, and/or the like, that are captured, recorded, sensed, detected, measured, or the like using smart biosensors, e.g., a heartrate monitor on a smart phone or smart watch.

Other external data that the machine learning module is trained on may include risk factors that are associated with the type of disease that is being diagnosed, e.g., lung or breast cancer. The risk factors may be related to the person's health, lifestyle, environmental conditions, socioeconomic status, employment, and/or the like. For example, the risk factors for lung cancer may include the patient's age, personal and family history of cancer, smoking history, size of nodule in the tissue, number of nodules in the tissue, characteristics of nodules in the tissue, location of nodules in the tissue, history of emphysema, body mass index, type and history of employment, where the patient has lived, and/or the like.

In another example embodiment, the risk factors for breast cancer may include age, genetic mutations, reproductive history, breast density, personal history of breast disease, family history of breast cancer, previous radiation therapy treatment, taking the drug diethylstilbestrol ("DES"), and/or the like.

Other external data that the machine learning module is trained on may include symptoms that are associated with the type of disease that is being diagnosed, e.g., lung or breast cancer. For example, the risk factors for lung cancer may include recent weight loss, blood in sputum, chest pain, cough, shortness of breath, wheezing, fatigue, bone pain, and/or the like. In another example embodiment, the risk factors for breast cancer may include lump size, lump growth, thickening of portion of the breast, dimpling of breast skin, flaky skin, pain in nipple, nipple discharge, changes in size and/or shape of breast, pain in breast, and/or the like.

The ML module 206, in certain embodiments, interfaces with a data store, or multiple data stores, to access the external patient data, which is collected from other patients and/or contains information the patient that is being diagnosed. A data store, for example, may be stored locally at a hospital or clinic, may be stored in the cloud behind a secure gateway that requires credentials to access, may be publicly accessible, and/or the like. In one embodiment, the ML module 206 periodically polls a data store to check for new data, receives a notification or signal that new data is available, and/or the like, which the ML module 206 uses to retrain and refine the machine learning model for the disease that is being diagnosed.

In one embodiment, the monitoring module 208 is configured to periodically, over time, measure the electrical impedance of the tissue of the patient's body between the interrogation electrode of the probe and the reference electrode and monitor progression of the disease in the tissue and effectiveness of treatment therapies in treating the disease in the tissue. For instance, the monitoring module 208 may track the patient's progress every day, every week, every month, and/or the like to determine whether a detected tumor is stable (indicating treatment is not having any effect), is getting bigger (indicating treatment is not working), is getting smaller (indicating treatment is working), and/or the like.

The monitoring module 208 may use the machine learning to generate suggestions, recommendations, and/or the like for treatment, lifestyle changes, and/or the like. The monitoring module 208 may also generate reports, that include trends, forecasts, analyses, and/or the like, based on the machine learning, that describe the patient's treatment, progression, and/or the like, also as it relates to other, similar patients that have similar biomarkers, risk factors, symptoms, diseases, nodule/tumor locations and sizes, and/or the like.

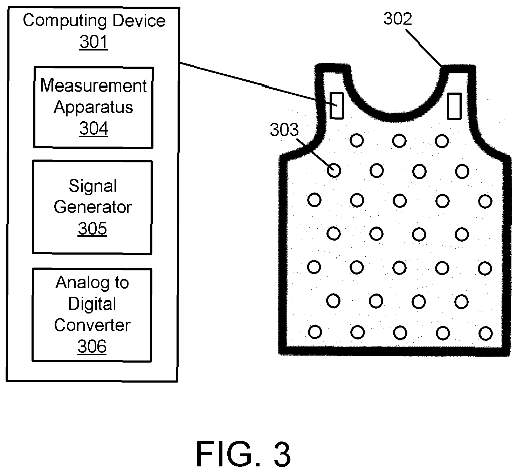

In one embodiment, the diagnostic apparatus 104 may be communicatively coupled to an electrode garment 212, as shown in Figure 3. Electrical impedance tomography has conventionally been used to create single slice images of the electrical impedance through the thorax, for example. This may be done using a circular array of electrodes placed on or around the chest to characterize the entire chest cavity. Electrical signals are induced through the electrodes, and an image is created using various mathematical algorithms.

As described herein, to facilitate efficient imaging and measuring of a patient using electrical impedance, in one embodiment, a garment 302, which may be substantially similar to the electrode garment 212 described above with reference to Figure 2, is configured with an array of electrodes 303, e.g., reference electrodes, that are placed in a predefined or random pattern on the garment 302. For instance, the electrodes 303 may comprise dorsally placed reference electrodes and ventrally placed signal electrodes.

In certain embodiments, the garment 302 may be comprised of two or more separate sheets or pieces of material, with each piece comprising electrodes 303 located in an array. The separate pieces may be stitched or otherwise fastened together so that the electrodes 303 can be used together or simultaneously. In certain embodiments, however, the electrodes 303 may be used independently, e.g., one at a time, in a specified pattern, e.g., every other electrode, and/or the like. The electrodes 303 may be permanently fastened or integrated into the garment 302 or selectively/removable attached to the garment 302, which allows electrodes 303 to be changed or replaced as needed.

In some embodiments, the electrodes 303 may be pre-treated with a conductive substance such as an electrode gel. In further embodiments, the electrodes 303 are positioned or located in a compliant pad of a conductive polymer. In various embodiments, the electrodes 303 are conductive elements that are connected by flexible routed conductors. In some embodiments, the electrodes 303 are embedded or integrated into a stretchable film or material.

The garment 302 may be a vest, as illustrated in Figure 3, but may also be embodied as a shirt, bra, cap, pants, underwear, belt, socks, headband, and/or other wearable material. The garment 302 may be made of various materials such as cotton, polyester, and/or the like. In certain embodiments, the garment 302 is reusable and can be washed with or without the electrodes 303. In other embodiments, the garment 302 is a one-time use garment that is disposable with or without the electrodes 303.

In one embodiment, the garment 302 includes a computing device 301 that includes a signal generator 305 and an analog to digital signal converter 306. The computing device 301 may be an off-the-shelf computing device such as a mobile device, a desktop or laptop computer, or a computing device that is specially configured and/or programmed to perform the functions/steps described herein. The signal generator 305 may generate electrical signals, e.g., AC, DC, high frequency signals, at a desired voltage. In certain embodiments, the electrodes 303 (on one side of the garment 302, e.g., the ventral side, and/or on both sides of the garment 302, e.g., the ventral and dorsal sides) are switchably connected to the signal generator 305.

In certain embodiments, the computing device 301, including a power source, e.g., a battery, the signal generator 305, analog to digital converter 306, and measurement apparatus 304 are built into the garment 302 so that the garment 302 and the computing device 301 are modular without requiring additional connectors, wires, power sources, etc. for connecting the garment 302 to an external computing device, e.g., computing device 150 and/or probe system 152 described above with reference to Figure 1C. In such an embodiment, the recorded or measured information is processed at the point of recording, wirelessly communicated to an external device for processing, or connected to an external device via a wired connection, e.g., a USB connection, for processing.

In one embodiment, each electrode 303 is switchably connected to the analog to digital signal converter 306, and the analog to digital signal converter 306 is configured to detect and record the complex nature of the signal including frequency, impedance, and phase. In embodiments where the computing device 301 is an external device, the signal generator 305 and the analog to digital converter 306 may each be coupled to a controller or other hardware component on the garment 302, which may be connected to the electrodes 303 in parallel or in series and transmits signals to/from the garment 302 from/to the computing device 301.

In certain embodiments, the electrodes 303 can be used to monitor heart signals, or other biometric information, or switched to excite and measure the bioimpedance of a target area. In various embodiments, all the electrodes 303 are activated/excited at the same time, or only a subset of the electrodes 303 are activated to measure a specific target area, e.g., a lung or portion of a lung, a breast or portion of a breast.

In one embodiment, the measurement apparatus 304 processes signals and measurements from the electrodes 303 on the garment 302 to determine information for diagnosing a subject or patient that is wearing the garment 302 based on electrical impedances. For instance, the measurement apparatus 304 may calculate a diagnostic score based on the differentiation between normal verses abnormal impedance measurements of the thoracic area (e.g., based on impedance measurements previously acquired from the patient or other patients). In one embodiment, the measurement apparatus 304 dynamically determines and selects a signal node/electrode 303 to excite based on algorithmic calculations, e.g., based on previous readings or measurements.

In various embodiments, the measurement apparatus 304 may be embodied as a hardware appliance that can be installed or deployed on the computing device 301, on the garment 302, or the like. In certain embodiments, the measurement apparatus 304 may include a hardware device such as a secure hardware dongle or other hardware appliance device (e.g., a set-top box, a network appliance, or the like) that attaches to a device such as the computing device 301, or the like, either by a wired connection (e.g., a universal serial bus ("USB") connection) or a wireless connection (e.g., Bluetooth^{®}, Wi-Fi, near-field communication ("NFC"), or the like); that attaches to an electronic display device (e.g., a television or monitor using an HDMI port, a DisplayPort port, a Mini DisplayPort port, VGA port, DVI port, or the like); and/or the like. A hardware appliance of the measurement apparatus 304 may include a power interface, a wired and/or wireless network interface, a graphical interface that attaches to a display, and/or a semiconductor integrated circuit device as described below, configured to perform the functions described herein with regard to the measurement apparatus 304.

The measurement apparatus 304, in such an embodiment, may include a semiconductor integrated circuit device (e.g., one or more chips, die, or other discrete logic hardware), or the like, such as a field-programmable gate array ("FPGA") or other programmable logic, firmware for an FPGA or other programmable logic, microcode for execution on a microcontroller, an application-specific integrated circuit ("ASIC"), a processor, a processor core, or the like. In one embodiment, the measurement apparatus 304 may be mounted on a printed circuit board with one or more electrical lines or connections (e.g., to volatile memory, a non-volatile storage medium, a network interface, a peripheral device, a graphical/display interface, or the like). The hardware appliance may include one or more pins, pads, or other electrical connections configured to send and receive data (e.g., in communication with one or more electrical lines of a printed circuit board or the like), and one or more hardware circuits and/or other electrical circuits configured to perform various functions of the measurement apparatus 304.

The semiconductor integrated circuit device or other hardware appliance of the measurement apparatus 304, in certain embodiments, includes and/or is communicatively coupled to one or more volatile memory media, which may include but is not limited to random access memory ("RAM"), dynamic RAM ("DRAM"), cache, or the like. In one embodiment, the semiconductor integrated circuit device or other hardware appliance of the measurement apparatus 304 includes and/or is communicatively coupled to one or more non-volatile memory media, which may include but is not limited to: NAND flash memory, NOR flash memory, nano random access memory (nano RAM or "NRAM"), nanocrystal wire-based memory, silicon-oxide based sub-10 nanometer process memory, graphene memory, Silicon-Oxide-Nitride-Oxide-Silicon ("SONOS"), resistive RAM ("RRAM"), programmable metallization cell ("PMC"), conductive-bridging RAM ("CBRAM"), magneto-resistive RAM ("MRAM"), dynamic RAM ("DRAM"), phase change RAM ("PRAM" or "PCM"), magnetic storage media (e.g., hard disk, tape), optical storage media, or the like.

In one embodiment, the measurement apparatus 304 interfaces with, is communicatively coupled to, and/or the like the diagnostics apparatus 104 to generate and apply electrical currents to the electrodes 303 in the garment 302, either individually, all together, for a specific area, and/or the like. In one embodiment, an interrogation electrode of a probe 160 may be applied to the patient's body, e.g., over the garment 302, and various electrical impedance measurements may be read, detected, taken, or the like from various reference electrodes 303 on the garment 302.

In certain embodiments, the ML module 206 generates recommendations, suggestions, directions, and/or the like for selecting reference electrodes on the garment 302 to use for measuring electrical impedances. For example, the ML module 206 may receive the current locations of the reference electrodes 303 that are being used and their measured electrical impedances, and based on the measured electrical impedances, the disease that is being diagnosed, and/or the like, the ML module 206, based on a trained ML model, may calculate, generate, determine, and/or the like an amount of current or pressured to be applied, the reference electrodes 303 to use on the garment 302, and/or the like.

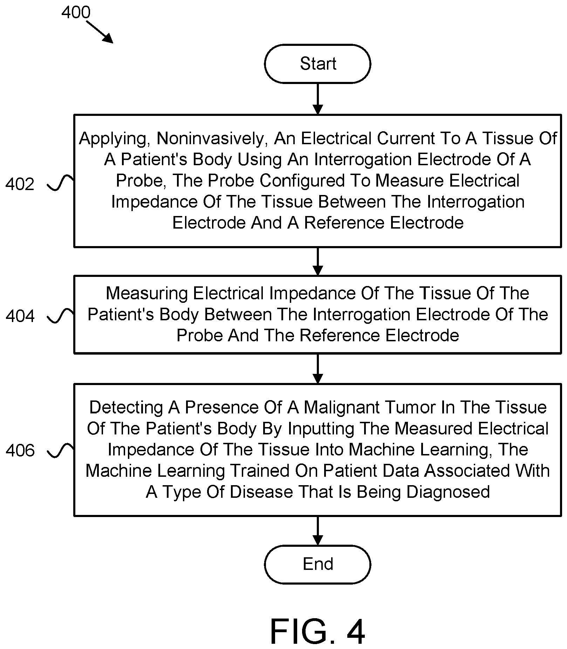

Figure 4 depicts a schematic flow chart diagram of one embodiment of a method 400 for noninvasive medical diagnostics using electrical impedance metrics and clinical predictors. In one embodiment, the method 400 begins and applies 402, noninvasively, an electrical current to a tissue of a patient's body using an interrogation electrode of a probe. The probe may be configured to measure electrical impedance of the tissue between the interrogation electrode and a reference electrode.

In further embodiments, the method 400 measures 404 electrical impedance of the tissue of the patient's body between the interrogation electrode of the probe and the reference electrode. In one embodiment, the method 400 detects 406 a presence of a malignant tumor in the tissue of the patient's body by inputting the measured electrical impedance of the tissue into machine learning. The machine learning may be trained on patient data associated with a type of disease that is being diagnosed, and the method 400 ends. In some embodiments, the current module 202, the measurement module 204, and/or the ML module 206 perform the various steps of the method 400.

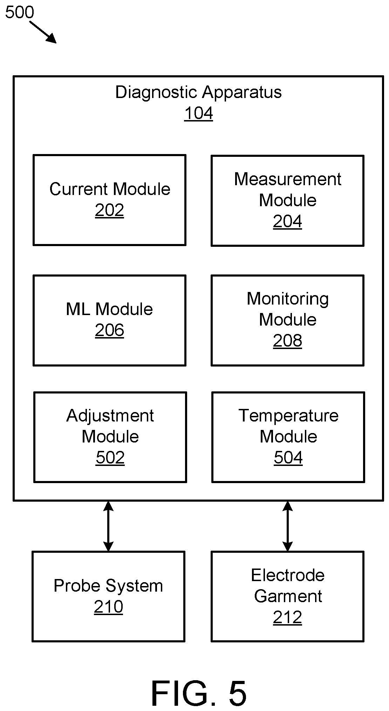

Figure 5 depicts one embodiment of an apparatus 500 for noninvasive medical diagnostics using electrical impedance metrics and clinical predictors. In one embodiment, the apparatus 500 includes an embodiment of a diagnostic apparatus 104. In one embodiment, the diagnostic apparatus 104 includes one or more of a current module 202, a measurement module 204, an ML module 206, and a monitoring module 208, which may be substantially similar to the current module 202, the measurement module 204, the ML module 206, and the monitoring module 208 described above with reference to Figure 2. In further embodiments, the diagnostic apparatus 104 includes an instance of an adjustment module 502 and/or a temperature module 504. In some embodiments, a probe system 210 and an electrode garment 212 are communicatively coupled to the diagnostic apparatus 104.

The adjustment module 502, in one embodiment, is configured to adjust a location of the probe on the patient's body according to feedback based on the measured electrical impedance of the tissue. For example, after an electrical current has been applied to a location on the patient's body using the interrogation electrode of the probe, and an electrical impedance has been measured, the adjustment module 502 may provide feedback indicating whether the measurement was a good or bad reading for the tissue that is being examined and/or for the disease that is being diagnosed.

In one embodiment the adjustment module 502 provides feedback to a user, e.g., the probe operator, that includes instructions for moving the probe to a different location (e.g., move up, down, left, right, between fingers 1 and 2, to location FML-8aR, and/or the like) to get a different impedance measurement, adjusting the angle of the probe (e.g., angle more towards the patient, place at a 45 degree angle, or the like), adjusting the pressure applied to the skin surface (e.g., increase or decrease pressure by a certain amount), adjusting the amount of electrical current that the probe applies (e.g., increase or decrease the voltage or current by a certain amount), and/or the like.

In certain embodiments, the adjustment module 502 provides feedback in real-time as the probe is moved around a surface of the user's skin and electrical current is applied to the patient's body using the probe. The adjustment module 502, for instance, may provide a visual representation of the part of the body where the probe is located and may show on a display the probe, or a graphical representation of the probe, in real-time being moved around that part of the body. The feedback may also display text instructions, videos, animations, and/or the like for adjusting the probe, the location of the probe, the settings of the probe, and/or the like to assist the user is determining an optimal location and/or settings for the disease or tissue of interest.

The adjustment module 502 may show a heatmap on the visual representation of the body around the area where the probe is located on the body that illustrates the different electrical impedances that are being measured and shows other locations where the different impedances are known to be located. For instance, if the user is looking for parts of the body with low impedances, e.g., lymphatic channels, on a patient's hand, the heatmap may use a color gradient, e.g., from red to green, that indicates areas of high impedances (red) to areas of low impedance (green), for both current impedance measurement and also known or precalculated, predefined impedances (e.g., from previous measurements, from other patients, or the like).

Thus, the feedback may include visual feedback, as described above, and/or audible feedback. The audible feedback may include voice commands, instructions, directions, tones (e.g., different sounds that indicate a good measurement, location, or angle v. a bad measurement, location, or angle), and/or the like for moving or adjusting the probe to place the probe in an optimal location for measuring electrical impedances based on the type of tissue or disease that the user wants to analyze. For example, the optimal location may be different for locating lymphatic channels than for locating nodules or tumors in a lung or breast.

In one embodiment, the adjustment module 502 provides feedback that the probe is not generating usable data, e.g., is not getting accurate, correct, defined, consistent, and/or the like electrical impedance measurements or readings. In such an embodiment, the adjustment module 502 may provide a message, visual or audible, that the probe is not generating usable data and may provide instructions or directions, visually and/or audibly, for adjusting the location, angle, pressure, voltage, current, and/or other settings of the probe to generate usable data.

In certain embodiments, the adjustment module 502 uses machine learning or other artificial intelligence to estimate or determine optimal locations, angles, pressures, currents, or other settings for the probe for measuring the patient's body based on the disease and/or tissue that is being analyzed using historical and current electrical impedance measurements. For example, the current probe settings and impedance measurements, disease of interest, tissue type, and/or the like may be input into a machine learning model that is trained using historical probe settings, impedance measurements, and/or the like for the disease/tissue of interest to determine, calculate, predict, or the like the optimal settings/location for the probe on the patient's body.

In one example embodiment, the diagnostic apparatus 104, including the adjustment module 502, measures the lymphatic system by using dielectric measurements on the skin surface. The dielectric measurements may offer valuable information by detecting diseases occurring within the body that are undetected and frequently prior to symptoms. When a disease is present, the lymphatic system acts a network of tissues and organs that help rid the body of toxins, waste, and other unwanted materials. The primary function of the lymphatic system is to transport lymph, a fluid containing infection-fighting white blood cells, throughout the body. It also collects any cancer cells if these are present. This lymph fluid then drains into the lymph vessels.

The diagnostic apparatus 104 is configured to locate the lymphatic system channels on the skin surface and also obtain bioconductance measurements that correlate with diseases. The human body is very complex, consisting of 11 systems including the lymphatic muscular, skeletal, nervous, circulatory, etc. Accessing the lymphatic system for a bioconductance measurement underneath the skin is invasive and locating the lymphatic system channels on the surface of the skin is extremely challenging as different body types make it difficult to perform a uniform measurement approach based on physical anatomical landmarks.

For example, cancer is a life-threatening disease that is difficult to diagnose as it can exist within the body without the manifestation of symptoms. However, if cancer is detected at an early stage it can be treated and individuals can potentially be cured of cancer. Conventional systems for measuring the lymphatic system suffer from a number of drawbacks - invasive measurements performed underneath the skin, difficult to locate the lymphatic channels/vessels below the skin, difficult to obtain reliable and repeatable measurements of the lymphatic system that may offer valuable diagnostic information, etc.

The diagnostic apparatus 104 described above, and more particularly the adjustment module 502, provides solutions for these drawbacks by non-invasively measuring the lymphatic system on the surface of the skin by identifying the correct location and angle on the skin surface to perform a measurement by identifying the location with the least amount of resistance in ohms and by providing visual and operator feedback as operator scans target area. Furthermore, the probe motor mitigates operator pressure and aborts inconsistent measurements. The diagnostic apparatus 104 performs multiple measurements and identifies inaccurate measurements based on averages and/or outlier identifier techniques.

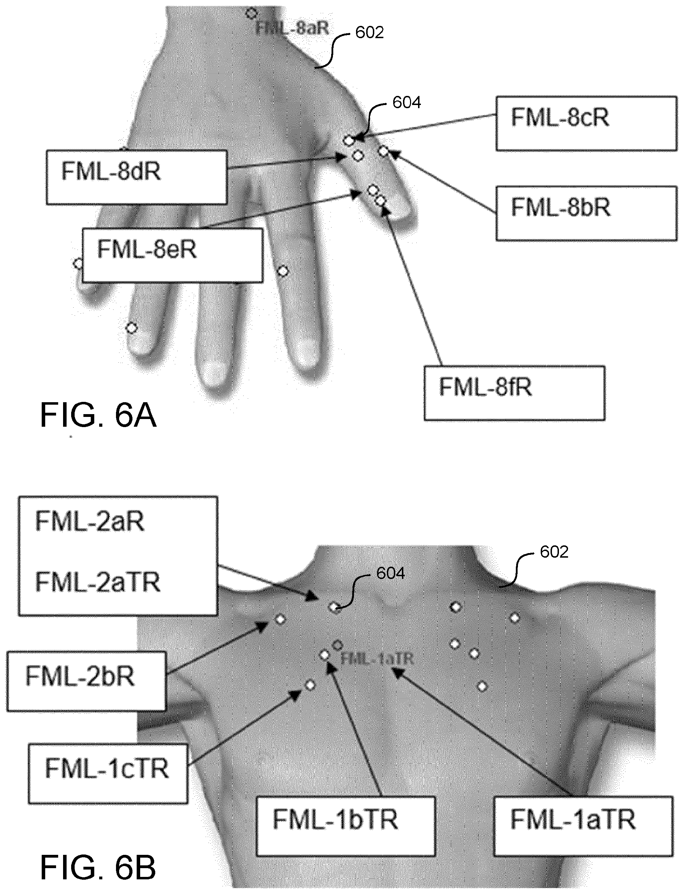

Figures 6A and 6B display embodiments of visual feedback that the adjustment module 502 provides on a display. In one example embodiment, during operation, the adjustment module 502 displays anatomical schematics on the display to guide the operator to the correct anatomical locations 604 on the subject 602. For example, to locate the lymphatic channel on a patient's hand, the operator first follows the screen prompt on the display that provides a visual display with anatomical references in the description for placing interrogation and/or reference electrodes at the following locations:
FML-8aR
Point Location - This point is located between the radius and navicular bones on the ulnar side of the extensor pollicis longus tendon.
Electrode Cable Location - Left Hand
FML-8bR
Point Location - This point is located at the distal diaphyseal end of the proximal phalanx of the thumb on its radial side. It is measured on a 45 degree angle with the probe pointing distally.
Electrode Cable Location - Left Hand
In another example, shown in Figure 6B, to locate the lymphatic channel on a patient's chest, the operator first follows the screen prompt on the display that provides a visual display with anatomical references in the description for placing interrogation and/or reference electrodes at the following locations:
FML-1aTR
Point Location - This point is located on the 2nd rib approximately 2 1/2 thumb widths lateral from the midline or depression point on the sternum.
Electrode Cable Location - Upper Right Back
FML-1bTR
Point Location - This point is located in the 2nd intercostal space on a line between the lateral insertion of the sternocleidomastoid muscle and the nipple. It is approximately 3-3 1/2 thumb widths from the midline.
Electrode Cable Location - Upper Right Back
FML-1cTR
Point Location - This point is located in the 3rd intercostal space approximately 3 1/2 thumb widths lateral from the middle of the chest.
Electrode Cable Location - Upper Right Back
FML-2aTR
Point Location - This point is located in the depression on the lower border of the clavicle, 2 thumb-widths lateral to the midline. The 2 thumb-width line is located midway between the midline and the mamillary line.
Electrode Cable Location - Lower Right Back
FML-2aR
Point Location - This point is located in the depression on the lower border of the clavicle, 2 thumb-widths lateral to the midline. The 2 thumb-width line is located midway between the midline and the mamillary line.
Electrode Cable Location - Left Hand
FML-2bR
Point Location - This point is located on the lateral aspect of the chest, in the first intercostal space, 6 thumb-widths lateral to the midline, 1 thumb-width inferior to FML-2cR.
Electrode Cable Location - Left Hand

To ensure that the angle and the location is correct, the device operator may hold down a button on the probe to initiate the lymphatic channel locator mode. The operator applies moisture to the measurement target region and then slides the probe tip, e.g., the interrogation electrode, back and forth listening to an audible tone or may follow a visual display (e.g., a heatmap) that identifies when the probe tip is at the correct location and/or angle with the least amount of resistance/impedance (e.g., the least amount of measured Ohms).

The table below illustrates the importance of identifying the correct location. The table shows measurements at four different measurement locations where each measurement shows the minimum ohms and maximum ohms obtained for a particular location. The adjustment module 502 allows the operator to identify the exact location where the measured impedance has the least amount of resistance (column 2). This function is significant as the resistance can vary, e.g., by about ~ 224% based on the operator's placement of the probe location and angle of the probe tip. The adjustment module 502 may provide feedback based on the information in the table in real-time to the operator, by audible tone or visually on the display, e.g., as raw data, as a heatmap, and/or the like.

**Table 1. Data showing impedance variance for four measurement locations.**

| Measurement Location | Ohms Min | Ohms Max | Ohms difference | % increase in Ohms |
|---|---|---|---|---|
| 1 | 32,000 | 118,000 | 86,000 | 269 |
| 2 | 52,000 | 178,000 | 126,000 | 242 |
| 3 | 52,000 | 118,000 | 66,000 | 127 |
| 4 | 79,000 | 282,000 | 203,000 | 257 |
| | | | Average | 224 |

The diagnostic apparatus 104 obtains bioconductance measurements by passing a current, e.g., of less than 25 microamps between reference electrodes placed on the subject's body, e.g., on the subject's back or hands and the probe (the interrogation electrode), which may be placed on the subject's chest, shoulders, and/or arms. The adjustment module 502 provides real-time visual and auditory feedback to the operator for quality assurance purposes, e.g., for moving the probe to a different location, for adjusting the angle, pressure, current, or the like, of the probe, and/or the like. The conductance measurement profile may be displayed visually on the display.

The diagnostic apparatus 104, may sample conductance values 25 times per second and monitor probe pressure to obtain accurate and consistent measurements. The diagnostic apparatus 104 monitors and controls probe pressure during measurements. After the measurement session is complete, the diagnostic apparatus 104 stores the data for processing by a classifier algorithm, a machine learning algorithm, and/or the like. The device classifier algorithm combines the measurement data into a composite risk score that corresponds with either a high or low likelihood of malignancy based on a predetermined score cut-off or threshold. After the data is processed using the algorithm, a report is generated indicating the health status of the patient.

In one practical example embodiment, use of the probe is non-invasive, with no exposure to radiation, and can typically be completed in 20-40 minutes. With the subject patient, typically dressed in a hospital gown, in a seated position, the operator enters the relevant user and patient information. The operator opens the single-use test kit. Single-use diaphoretic electrodes are applied to specific locations on the back and hands of the subject as described in the operator's manual and as demonstrated during operator training. Following onscreen prompts that the adjustment module 502 provides, the operator uses the probe to acquire measurement data from areas of the body associated with diseases (e.g., cancers) or tissues that are being analyzed, e.g., the chest, shoulders, and arms of the subject.

While in use, the adjustment module 502 provides onscreen written descriptions, images, prompts, directions, instructions and/or the like for each point of measurement in real-time as the test is being performed. The operator observes real-time monitoring, validation, and recording of each measurement. Should re-measurement be required, the adjustment module 502 provides a visual and/or audible notification that it has not received usable data whereupon the operator can remeasure. The probe apparatus 104 saves the data for further use and analysis, e.g., as part of a machine learning algorithm, engine, model, training, and/or the like.

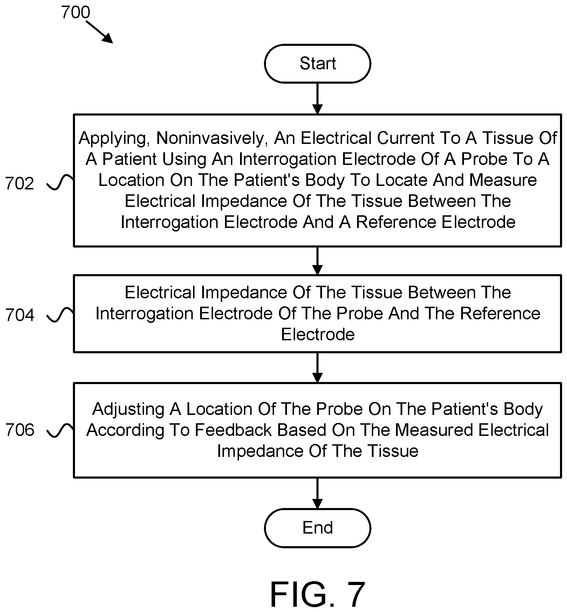

Figure 7 depicts a schematic flow chart diagram of one embodiment of a method 700 for noninvasive medical diagnostics using electrical impedance metrics and clinical predictors. In one embodiment, the method 700 begins and applies 702, an electrical current to a tissue of a patient using an interrogation electrode of a probe to a location on the patient's body to locate and measure electrical impedance of the tissue between the interrogation electrode and a reference electrode.

In further embodiments, the method 700 measures 704 electrical impedance of the tissue between the interrogation electrode of the probe and the reference electrode. In one embodiment, the method 700 adjusts 706 a location of the probe on the patient's body according to feedback based on the measured electrical impedance of the tissue, and the method 700 ends. In some embodiments, the current module 202, the measurement module 204, and/or the adjustment module 502 perform the various steps of the method 700.

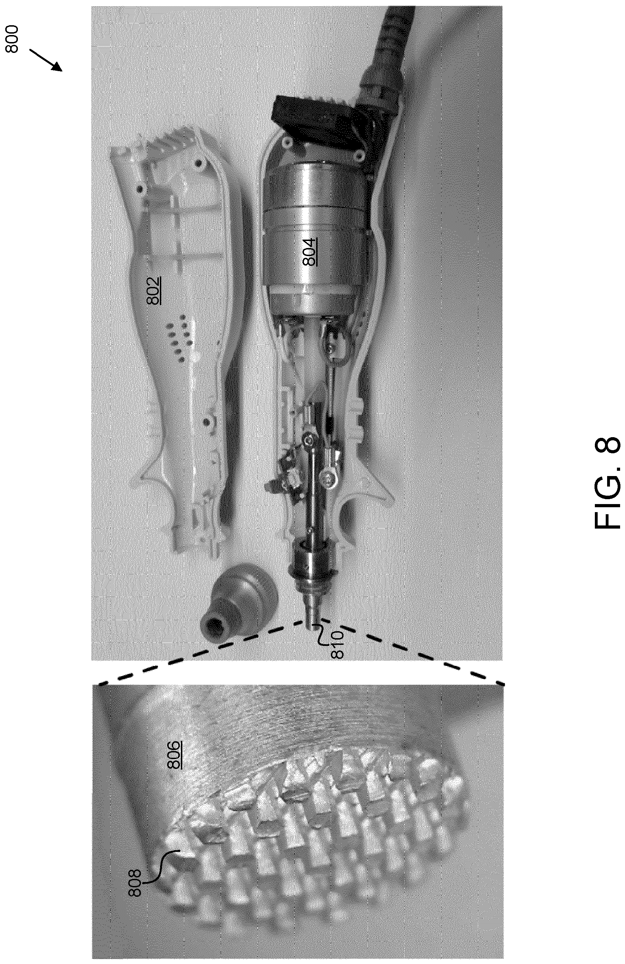

Figure 8 depicts one embodiment of an impedance measurement device 800 in accordance with the subject matter described herein. In one embodiment, the device 800 includes an operator-held probe housing 802 through which a linear voice coil motor 804 controls the position and force applied by an interrogation electrode tip 806. In one embodiment, the interrogation electrode tip 806 is centrally located coaxially with the probe tip 810 and is selectively coupleable to the probe tip 810, e.g., using a threaded attachment, using magnets, using a snap fit, using a friction fit, and/or the like. In one embodiment, the interrogation electrode tip 806 is disposable. In such an embodiment, the interrogation electrode tip 806 is surrounded by an annular shroud and is configured to extend from the probe tip 810 to apply a force to the surface of a patient's body.

In one embodiment, the interrogation electrode tip 806 has a textured surface that includes a plurality of protrusions 808. The protrusions 808, in one embodiment, may be any size (e.g., length and/or width) and shape. In one embodiment, the protrusions 808 have a hexagonal shape.

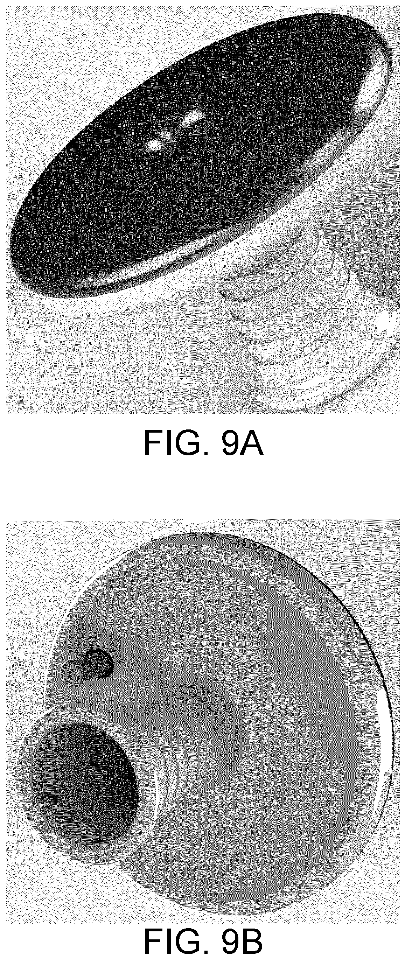

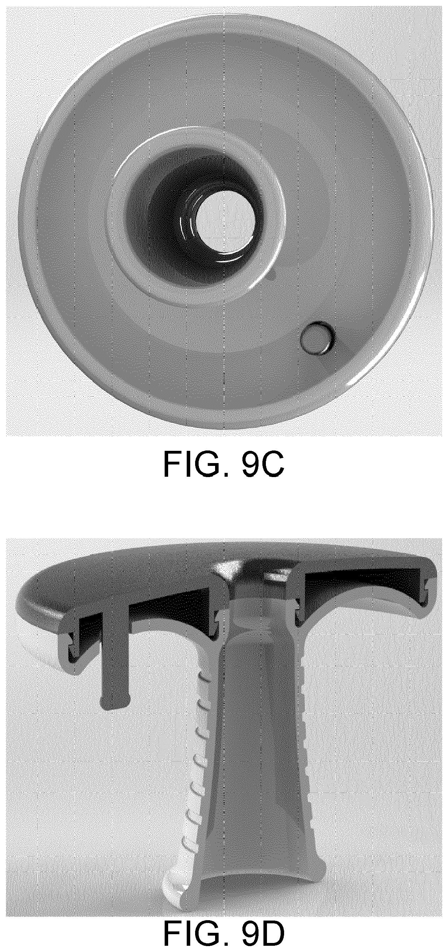

In one embodiment, the interrogation electrode tip 806 has a disc shape, shown in Figures 9A-9D, that has a substantially smooth surface. In some embodiments, the interrogation electrode has a diameter of within a range of 4.0 mm to 5.0 mm. In certain embodiments, the protrusions 808 have a diameter within a range of 0.5 mm and 0.6 mm. In one embodiment, the interrogation electrode tip 806 is made of brass, silver-silver chloride, gold, stainless steel, and/or the like.

In one embodiment, the interrogation electrode tip 806 can be heated or cooled, as described below, to a predetermined temperature that corresponds with a predefined electrical conductance level. In such an embodiment, the probe tip 210 may include a heating element and/or a cooling element that is used to control the temperature of the interrogation electrode tip 806.

In one embodiment, the impedance measurement device 800 is part of a probe system, such as the probe system 152 described above with reference to Figure 3. Thus, the impedance measurement device 800 may be connected to or may otherwise be in communication with a reference electrode and a computer assembly, such as the reference electrode 162 and computer assembly 150 described above with reference to Figure 1C.

In an example embodiment, the impedance measurement device 800, e.g., the handheld probe, has an overall length of approximately 18.5 cm with a maximum diameter of 4 cm with a weight of 280 g. Inside the probe handle, in one embodiment, is a conductive shaft driven by a voice coil linear motor, and a cooling fan. The shaft is threaded, in one embodiment, and is attached by the operator to the textured disposable tip, e.g., the interrogation electrode tip 206. During operation, in one embodiment, the device 800 applies a nominal force of 5.5 N to the interrogation electrode tip 206 onto the skin. The operator, in one embodiment, pushes the interrogation electrode tip 206 onto the skin with a force that must exceed this probe force. The interrogation electrode tip 206, in one embodiment, is surrounded by a small annular shroud. The operator pushes and holds the outer annular tip flush with the skin while the coaxially located electrode automatically extends and increases the force to the set level.

In one embodiment, the system measures the resistance between the location on the body that the operator places the interrogation electrode tip 206, and the handheld brass electrode, e.g., the reference electrode 162 depicted in Figure 1C. The device 800, in one embodiment, begins recording as soon as the interrogation electrode tip 206 is touched to the skin. Simultaneously, in one embodiment, the voice coil motor algorithm is activated or triggered, and the interrogation electrode tip 206 force increases in a controlled ramp up to the control level of 5.5 N. The device 800 monitors the signal resistance and holds the interrogation electrode tip 206 in place for a controlled time based on the stability of the signal. This time, for example, generally takes between 7 and 10 seconds. At the end of the signal acquisition period, in one embodiment, the probe tip motor is deactivated, and signal recording is terminated, and then the operator moves the interrogation electrode tip 206 to measure the next predefined anatomical location, moistens the skin as detailed in the protocol, and takes the next measurement.

Referring to Figure 5, in one embodiment, the current module 202 is configured to apply an electrical current to at least one interrogation electrode tip 206 placed on a surface of a person's body within a Sappey Plexus region of the person's breast. As used herein, the Sappey Plexus region of a person's breast comprises the area of a breast that includes a network of lymphatics in the areola of the nipple.

In one embodiment, the measurement module 204 is configured to measure an electrical impedance of the person's tissue between the at least one interrogation electrode 206 placed within the Sappey Plexus region of the person's breast and a reference electrode 162.

In one embodiment, the measurement module 204 is further configured to compare the measured electrical impedance to previously-captured electrical impedance measurements of corresponding tissue to determine an indication of a presence of a malignant tumor in the person's tissue.

In one embodiment, the previously-captured electrical impedance measurements of the corresponding tissue comprise electrical impedance measurements of tissue from within the Sappey Plexus region of different people, e.g., data from other patients that are free of tumors, that have benign tumors, and/or that have malignant tumors. In further embodiments, the previously-captured electrical impedance measurements of the corresponding tissue comprise electrical impedance measurements of tissue from within the Sappey Plexus region of the person's other breast, which may be free of tumors, have benign tumors, and/or have malignant tumors.

In one embodiment, the ML module 206 is configured to provide the measured electrical impedance to a machine learning model that is trained on previously-measured electrical impedances, risk factors, and/or patient data for other people who have been diagnosed with benign and malignant tumors to calculate a risk score for the person. In one embodiment, the risk score may comprise a rank, value, rating, percentage, probability, likelihood, and/or the like of the patient having a malignant tumor, a benign tumor, or no tumor within the measured area, e.g., the Sappey Plexus area, of the patient's body.

In one embodiment, the ML module 206 is configured to receive mammogram information associated with the person's breast. In such an embodiment, the mammogram information is analyzed, e.g., by the ML module 208, to determine whether the mammogram information indicates a presence of a nodule within the person's breast. If so, in one embodiment, the ML module 206 inputs the mammogram information into the machine learning model to further calculate the risk score for the person based on the measured electrical impedance.

In one embodiment, the ML module 206 is configured to periodically update the person's risk score based on new, updated, revised, changed, adjusted, modified, and/or the like electrical impedance measurements and changes in risk factors and/or patient data (described above), e.g., the patient started or stopped smoking or drinking, it is determined that the patient's family has a history of breast cancer, the patient's age or weight has changed, and/or the like. The ML module 206 may provide or input the new information, in one embodiment, may be input or provided to the machine learning model to determine an effectiveness of treatment for post treatment monitoring, e.g., to determine if the patient's tumor has grown or shrunk, to determine if the patient's symptoms have decreased or increased, and/or the like in response to a medication, dosage, duration, and/or the like.

In one embodiment, the temperature module 504 is configured to heat the interrogation electrode tip 206 to a temperature that corresponds to a predefined electrical conductance. In such an embodiment, the temperature module 504 adjusts a temperature of the interrogation electrode tip 206 according to a controlled heat profile until a stable electrical current is detected between the at least one interrogation electrode and the reference electrode. The controlled heat profile, for example, may be a mapping of temperatures to electrical current and/or electrical impedances for a particular area of the patient's body, e.g., the Sappey Plexus area. In such an embodiment, the measurement module 204 is configured to capture electrical impedance measurements at various temperatures of the controlled heat profile. In one embodiment, the temperature module 504 heats the interrogation electrode tip 206 to the patient's body temperature.

Figures 9A-9D depict one embodiment of an interrogation electrode tip 206 in accordance with the subject matter described herein. In the depicted embodiment, the interrogation electrode tip 206 has a disc or circular shape that has a smooth surface. However, in some embodiments, the surface is a textured surface that has a plurality of protrusions 808, as described above. In certain embodiments, the interrogation electrode tip 206 selectively connects to a probe tip 810, e.g., using a threaded fit, a snap fit, a friction fit, a clip fit, and/or the like. In this manner, different disposable tips may be used, different tip types (e.g., tips of different materials, shapes, textures, and/or the like) may be used, and/or the like.

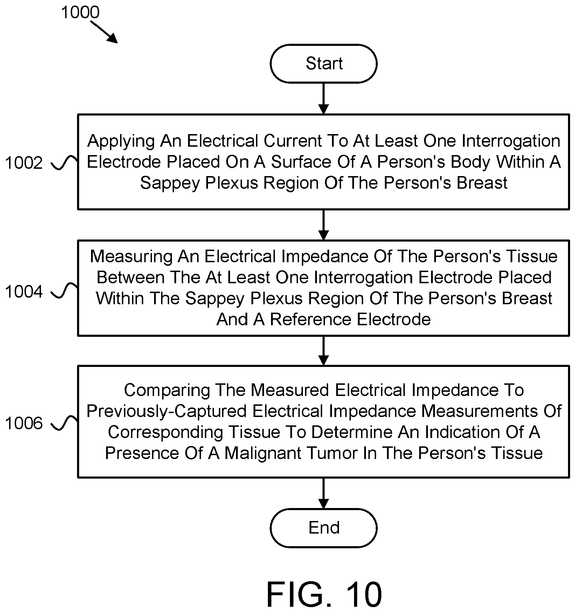

Figure 10 is a schematic flow chart diagram illustrating one embodiment of a method 1000 for noninvasive medical diagnostics using electrical impedance metrics and clinical predictors. In one embodiment, the method 1000 begins and applies 1005 an electrical current to at least one interrogation electrode placed on a surface of a person's body within a Sappey Plexus region of the person's breast.

In further embodiments, the method 1000 measures 1010 an electrical impedance of the person's tissue between the at least one interrogation electrode placed within the Sappey Plexus region of the person's breast and a reference electrode. In certain embodiments, the method 1000 compares 1015 the measured electrical impedance to previously-captured electrical impedance measurements of corresponding tissue to determine an indication of a presence of a malignant tumor in the person's tissue, and the method 1000 ends. In one embodiment, the current module 202 and the measurement module 204 perform the various steps of the method 1000.

A means for applying an electrical current to at least one interrogation electrode placed on a surface of a person's body within a Sappey Plexus region of the person's breast may include a diagnostic module 104, a current module 202, an impedance measurement device 800, an electrode probe, and/or the like.

A means for measuring an electrical impedance of the person's tissue between the at least one interrogation electrode placed within the Sappey Plexus region of the person's breast and a reference electrode may include a diagnostic module 104, a measurement module 204, an impedance measurement device 800, a computing device, a processor, a memory, and/or the like.

A means for comparing the measured electrical impedance to previously-captured electrical impedance measurements of corresponding tissue to determine an indication of a presence of a malignant tumor in the person's tissue may include a diagnostic module 104, a measurement module 204, an impedance measurement device 800, a computing device, a processor, a memory, and/or the like.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. An apparatus, comprising:
at least one interrogation electrode;
a reference electrode;
a processor; and
a memory that stores code executable by the processor to:
apply an electrical current to the at least one interrogation electrode placed on a surface of a person's body within a Sappey Plexus region of the person's breast;
measure an electrical impedance of the person's tissue between the at least one interrogation electrode placed within the Sappey Plexus region of the person's breast and the reference electrode; and
compare the measured electrical impedance to previously-captured electrical impedance measurements of corresponding tissue to determine an indication of a presence of a malignant tumor in the person's tissue.

2. The apparatus of claim 1, wherein the interrogation electrode is an electrode tip of an electrode probe.

3. The apparatus of claim 2, wherein the electrode tip has a disc shape and a substantially smooth surface.

4. The apparatus of claim 2, wherein the electrode tip comprises a textured surface, the textured surface of the electrode tip comprising a plurality of protrusions, each of the plurality of protrusions having a hexagonal shape.

5. The apparatus of any one of claims 2 to 4, wherein the electrode tip made of a material selected from the group comprising brass, silver-silver chloride, gold, and stainless steel.

6. The apparatus as claimed in any preceding claim, wherein the code is further executable by the processor to heat the at least one interrogation electrode to a temperature corresponding to a predefined electrical conductance.

7. The apparatus of as claimed in any preceding claim , wherein the code is further executable by the processor to adjust a temperature of the at least one electrode according to a controlled heat profile until a stable electrical current is detected between the at least one interrogation electrode and the reference electrode.

8. The apparatus of claim 7, wherein the code is further executable by the processor to capture electrical impedance measurements at various temperatures of the controlled heat profile.

9. The apparatus of as claimed in any preceding claim, wherein the previously-captured electrical impedance measurements of the corresponding tissue comprise electrical impedance measurements of tissue from within the Sappey Plexus region of at least one of different people and the person's other breast.

10. The apparatus of any preceding claim, wherein the code is further executable by the processor to provide the measured electrical impedance to a machine learning model that is trained on previously-measured electrical impedances, risk factors, and patient data for other people who have been diagnosed with benign and malignant tumors to calculate a risk score for the person.

11. An apparatus, comprising:
means for applying an electrical current to at least one interrogation electrode placed on a surface of a person's body within a Sappey Plexus region of the person's breast;
means for measuring an electrical impedance of the person's tissue between the at least one interrogation electrode placed within the Sappey Plexus region of the person's breast and a reference electrode; and
means for comparing the measured electrical impedance to previously-captured electrical impedance measurements of corresponding tissue to determine an indication of a presence of a malignant tumor in the person's tissue.

12. A method, comprising:
applying an electrical current to at least one interrogation electrode placed on a surface of a person's body within a Sappey Plexus region of the person's breast;
measuring an electrical impedance of the person's tissue between the at least one interrogation electrode placed within the Sappey Plexus region of the person's breast and a reference electrode; and
comparing the measured electrical impedance to previously-captured electrical impedance measurements of corresponding tissue to determine an indication of a presence of a malignant tumor in the person's tissue.

13. The method of claim 12, wherein the previously-captured electrical impedance measurements of the corresponding tissue comprise electrical impedance measurements of tissue from within the Sappey Plexus region of different people.

14. The method of claim 12, wherein the previously-captured electrical impedance measurements of the corresponding tissue comprise electrical impedance measurements of tissue from within the Sappey Plexus region of the person's other breast.

15. The method of as claimed in any one of claims 12 to 15, further comprising providing the measured electrical impedance to a machine learning model that is trained on previously-measured electrical impedances, risk factors, and patient data for other people who have been diagnosed with benign and malignant tumors to calculate a risk score for the person.

16. The method of claim 15, further comprising:
receiving mammogram information associated with the person's breast; and in response to determining that the mammogram information indicates a presence of a nodule within the person's breast, inputting the mammogram information into the machine learning to further calculate the risk score for the person based on the measured electrical impedance.

17. The method of claim 15, further comprising periodically updating the person's risk score based on updated electrical impedance measurements and changes in risk factors and patient data to determine an effectiveness of treatment for post treatment monitoring.

18. The method of as claimed in any one of claims 12 to 17, further comprising heating the at least one interrogation electrode to a temperature corresponding to a predefined electrical conductance.

19. The method of claim 18, further comprising adjusting a temperature of the at least one electrode according to a controlled heat profile until a stable electrical current is detected between the at least one interrogation electrode and the reference electrode.

20. The method of claim 19, further comprising capturing electrical impedance measurements at various temperatures of the controlled heat profile.
